# EUROPEAN PATENT SPECIFICATION

(11) **EP 2 111 833 B1**
(45) Date of publication and mention of the grant of the patent: **02.01.2013**
(21) Application number: 08706451.5
(22) Date of filing: 02.02.2008
(51) Int. Cl.: A61F 13/472, A61L 15/42, A61F 13/84

(54) **A FAR-INFRARED ANION COMPOUND SANITARY NAPKIN AND PAD**
FERNINFRAROT- UND ANIONEN-VERBUND-DAMENBINDE UND AUFLAGE DAFÜR
SERVIETTE HYGIÉNIQUE ET COUCHE CONTENANT UN COMPOSÉ ANIONIQUE DANS L'INFRAROUGE LOINTAIN

(30) Priority: 15.02.2007 CN 200720048763 U
(43) Date of publication of application: 28.10.2009
(73) Proprietor: Chen, Huaide, Guangzhou City Guangdong 510180 (CN)
(72) Inventor: Chen, Huaide, Guangzhou City Guangdong 510180 (CN)
(74) Representative: Stiebe, Lars Magnus
(86) International application number: PCT/CN2008/000267
(87) International publication number: WO 2008/098473

(56) References cited:
- WO-A1-2005/118010
- CN-A- 1 095 118
- CN-A- 1 098 318
- CN-A- 101 015 708
- CN-Y- 2 405 575
- CN-Y- 2 655 853
- CN-Y- 2 704 324
- CN-Y- 2 740 166
- FR-A1- 2 839 891
- JP-A- 2000 325 396
- JP-A- 2001 218 790
- US-A1- 2005 159 722
- US-A1- 2006 062 816
- US-A1- 2007 197 989

## Description

### FIELD OF THE INVENTION

The present invention relates to a sanitary napkin/pad, in particular to a far infrared anion complex sanitary napkin/pad.

### BACKGROUND OF THE INVENTION

According to investigation, a majority of women suffer from various degree of the gynecological inflammation. The genital itch is caused by easy infection of candida albicans, which is due to the hormonal change during menstruation that causes the decrease of the acidity in the vagina, on top of the impermeability of the sanitary napkin, and the poor air circulation in the vaginal area turning the vagina into a culture medium for bacteria. This symptom is worse during the sultry and humid weather. The menstruation also sends out unpleasant smell that greatly affects female's daily life and work. Therefore, the functions about anti-inflammation and deodorization for the sanitary napkin are gaining more and more public's attention. At present, regarding the solution for anti-inflammation and deodorization effects on the sanitary napkin, the most common practice is to add anti-inflammation medicine directly onto the sanitary napkin. However, when using this kind of sanitary napkin, the medicine directly affects the surrounding area of the vagina which may cause allergic reaction, some side effects or discomfort to the contacted area. The menstruation may also reduce the efficacy of the medicine. As a result, this kind of sanitary napkin is not suitable for using widely.

### SUMMARY OF THE INVENTION

Accordingly, it is an object of the present invention is to provide a far infrared anion complex sanitary napkin/pad with good effects of sterilization, anti-inflammation, and deodorization without irritation.

To achieve the above object, the present invention is carried out by the following technical solution:

A far infrared anion complex sanitary napkin/pad having multi-layer structure comprises, in sequence from upper to lower layer, a web layer, an absorbent layer, a waterproof coating layer and an adhesive layer, which are stuck firmly together. The sanitary napkin/pad further comprises a floral essential layer and a far infrared anion fiber layer attached together firmly. The floral essential layer is paper/fabric layer soaked with extracted floral essential solution; the far infrared anion fiber layer is woven of fiber obtained from a mixture of 8-10 wt% of anion mother grain, 8-10 wt% of far infrared mother grain, and homopolymer carrier or spinning liquid. The anion mother grain is made from nano grade mineral powder that activates anion in the air. The far infrared mother grain is made from nano grade mineral powder that activates far infrared.

Preferably, the nano grade mineral powder is nano grade tourmaline micro-powder that activates anions in the air. The nano grade mineral powder is nano grade far infrared ceramic micro-powderwhich activates far infrared. The far infrared anion fiber layer automatically releases anions and far infrared. Comparing the far infrared anion fiber with the conventional surface coating method, the far infrared produced by this kind of fiber has better durability. When the far infrared mother grain becomes humid upon contacting with human body, it releases the far infrared that penetrates the subcutaneous tissue of human body. The far infrared enhances human body's micro-circulatory system, the metabolism and the immunity. Anions also have good disinfecting effect. Negative oxygen ions have higher activity that can destruct the cell membrane of the bacterium or the activity of the protoplasm enzyme. Moreover, once the anions bond with the bacteria, the bacterial structure is changed, or the energy is converted which leads to bacteria death and disables the ability to produce new bacterin. Thus, it serves the purposes of disinfection and sterilization. Anion powder can ionize the air, and it also has the thermoelectric and piezoelectric characteristics. Therefore, under minor changes of temperature and pressure, it creates the voltage difference between anion crystals. The static voltage could be up to one million volts which will create an electric field. The high voltage ionizes the air in the electric field, and the electrons that are hit will stick to nearby water and oxygen molecules, which transform to air anions, which are negative hydrogen ion and negative oxygen ion. Both negative ions have the features of disinfection, sterilization, and prevention of many gynecological diseases. Anion lining has the advantages of softness, water resistance, and obvious effect of anion. Especially with added infrared emission material, the far infrared anion fibers can produce anion and infrared that will improve the blood circulation, aid in sleep, and enhance the health benefits of the product. Therefore, utilizing this technology on the sanitary napkin/pad will help to improve the micro-circulation around the vagina, to increase the amount of biological enzyme, to increase the acidic secretion in the vagina, and to adjust the PH value. All these effects are beneficial to female's health.

Preferably, the floral essential solution is made from a floral mixture of 3-8 parts by weight of lily, 8-14 parts by weight of honeysuckle flower, 3-8 parts by weight of chrysanthemumflower, 4-9 parts by weight of tree peony, 3-8 parts by weight of Chinese rose, 2-6 parts by weight of clove, 3-8 parts by weight of orange blossom, 1-6 parts by weight of common coltsfoot flower, 3-8 parts by weight of lotus, 8-16 parts by weight of jasmine flower, 5-10 parts by weight of rose, 6-10 parts by weight of common peony, 3-8 parts by weight of silktree albizzia flower, 3-6 parts by weight of wintersweet flower , 5-12 parts by weight of peppermint, 4-10 parts by weight of licorice, 3-8 parts by weight of mugwort leaf, 4-8 parts by weight of drug sweetflag rhizome, 3-12 parts by weight of lavender and 2-10 parts by weight of lemongrass. The floral mixture goes through extraction to obtain a blended essential oil; then the blended essential oil is diluted to 1000~1200 times to obtain the floral essential solution. The floral essential solution has the features of antibacterial, sterilization, and skin benefits. The floral essential solution also has the ability to increase the amount of oxygen that the anion material can carry, which is prominently beneficial to female's health.

The far infrared anion complex sanitary napkin/pad also includes a Chinese herbal essential layer. The Chinese herbal essential layer is paper/fabric layer soaked with Chinese herbal essential solution.

Preferably, the Chinese herbal essential solution is made from a mixture of Chinese herbs -containing 5-12 parts by weight of dried rehmannia root, 8-16 parts by weight of radix scrophulariae, 4-8 parts by weight of angelica, 8-16 parts by weight of honeysuckle flower, 5-12 parts by weight of forsythia fruit, 5-12 parts by weight of baicalin, 4-8 parts by weight of red paeony root, 5-12 parts by weight of tree paeony bark, 5-12 parts by weight of poria skin, 1-5 parts by weight of licorice , 6-15 parts by weight of Radix sophorae flavescentis, 12-18 parts by weight of common cnidium fruit, 12-18 parts by weight of belvedere fruit, 12-18 parts by weight of densefruit pittany root-bark, 4-8 parts by weight of pricklyash peel, 1-4 parts by weight of halite. The mixture of Chinese herbs is then cooked, filtered, and purified to obtain the Chinese herbal essential solution. The Chinese herbal essential solution has remarkable beneficial effects on female's outer genital itch, abnormal leucorrhea, and vaginitis.

Preferably, nano-silver and bamboo vinegar or bamboo charcoal are added into the absorbent layer. The addition includes 0.01-5 parts by weight of nano-silver having diameter less than 20nm, 0.1-10 parts by weight of bamboo vinegar or 0.1-10 parts by weight of bamboo charcoal. The amount of the nano-silver on the absorbent layer is 1∼400µg/cm². The nano-silver has the characteristics of durable effects, wide spectrum, strong antibacterial effect, better stabilization, better effect upon interacting with water, non-toxic, and no pollution to the environment. The more important is that it has the ability to release far infrared, which would work with the far infrared anion fiber layer in the sanitary napkin/pad to improve the effect of sterilization, anti-inflammation and deodorization. The ethanol material in the bamboo vinegar has the features of skin cleansing and sterilization. Therefore, the bamboo vinegar has the benefits of sterilization, itch relief, and deodorization. The acetic acid, ethyl ether, and alcohol in the bamboo charcoal have the benefits of deodorization, disinfection, and moisture absorption.

Preferably, the paper/fabric layer is a tissue layer, a non-woven fabric layer or a puffy cotton layer.

Preferably, the adhesive layer is stripe interrupted adhesive layer, which improves airflow.

Preferably, the floral essential layer is above the Chinese herbal essential layer, which can avoid the stimulation of the Chinese herbal essential layer to the skin of human body.

Preferably, the floral essential layer is tightly attached to the far infrared anion fiber layer, and the essential liquid in cooperating with the anion materials improves the oxygen content.

Preferably, the floral essential layer is above the far infrared anion fiber layer, the floral smell is emitted by the floral essential layer, and the floral essential layer with health effect is suitable to touch with the skin.

Preferably, the Chinese herbal essential layer is located at the central portion of the sanitary napkin/pad, and the ratio of the length and the width of the Chinese herbal essential layer with respect to the length and the width of the sanitary napkin/pad is in a range of 1/2 to 2/3, and thus the materials are concentrated and saved.

The far infrared anion complex sanitary napkin may be provided with two wings on the two side edges, and the far infrared anion complex sanitary pad may be also provided with or without two wings on the two side edges.

The nano grade means that the diameter is not more than one billionth of a meter.

The advantages of present invention are: (1) the floral essential solution not only has the features of sterilization, anti-inflammation, and skin benefits, but also blocks the medicine in the lower layer to directly irritate the contacted skin; (2) the floral essential layer sticking firmly with the far infrared anion fiber layer has the ability to increase the amount of oxygen that anion material can carry, which is beneficial to female's health; (3) the Chinese herbal essentialsolution has a remarkable beneficial effect on female's outer genital itch, abnormal leucorrhea, and vaginitis; (4) the far infrared mother grain in the far infrared anion fiber layer facilitates the micro-circulation around the vagina, increases the amount of biological enzyme and the acidic secretion in the vagina, adjusts the PH value, and increases the immunity of female's body. The anion launched from anion mother grain has the features of disinfection, sterilization, and prevents many gynecological diseases. (5) The additional nano-silver and bamboo vinegar or bamboo charcoal in the absorbent layer have the features of sterilization, anti-inflammation, and deodorization. The effect would be greatly enhanced when using with the far infrared anion fiber layer in the sanitary napkin/pad; (6) when comparing the far infrared anion fiber with the conventional surface coating method, the fiber producing far infrared has better durability.

### BRIEF DESCRIPTION OF THE DRAWINGS

Fig. 1 is a schematic view of the sanitary napkin/pad according to a preferred embodiment of the present invention;
Fig. 2 is a schematic cross-sectional view of the far infrared anion complex sanitary napkin/pad of Embodiments 1, 3 and 5;
Fig. 3 is a schematic cross-sectional view of the far infrared anion complex sanitary napkin of Embodiments 2, 4 and 6;
Fig. 4 is a schematic cross-sectional view of the far infrared anion complex sanitary napkin/pad of Embodiment 7;

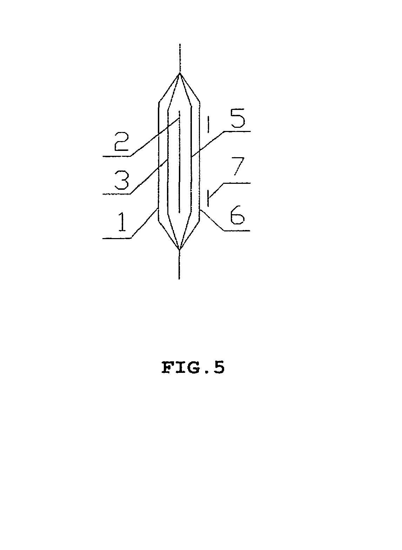

Fig. 5 is a schematic cross-sectional view of the far infrared anion complex sanitary napkin/pad of Embodiment 8.

### DETAILED DESCRIPTION OF THE INVENTION

### Embodiment 1:

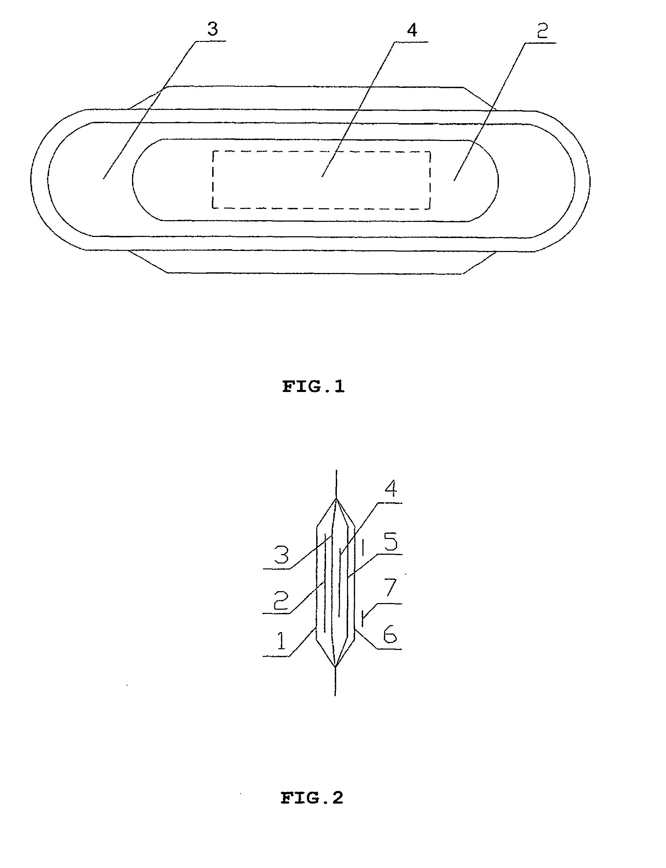

Referring to Figs. 1 and 2, a multi complex structure of the far infrared anion complex sanitary napkin/pad is shown. From upper to lower layer, the sanitary napkin/pad includes in sequence a web layer 1, a floral essential layer 2, a far infrared anion fiber layer 3, a Chinese herbal essential layer 4, an absorbent layer 5, a waterproof coating layer 6 and an adhesive layer 7. All layers are stuck firmly together.

The floral essential layer 2 is non-woven fabric soaked with the extracted floral essential solution. The floral essential solution is made from a floral mixture containing 5 parts by weight of lily, 9 parts by weight of honeysuckle flower, 4 parts by weight of chrysanthemum flower , 6 parts by weight of tree peony, 5 parts by weight of Chinese rose, 2 parts by weight of clove, 4 parts by weight of orange blossom, 5 parts by weight of common coltsfoot flower, 6 parts by weight of lotus, 10 parts by weight of jasmine flower, 8 parts by weight of rose, 7 parts by weight of common peony, 4 parts by weight of silktree albizzia flower , 5 parts by weight of wintersweet flower , 8 parts by weight of peppermint, 6 parts by weight of licorice, 5 parts by weight of mugwort leaf, 6 parts by weight of drug sweetflag rhizome, 8 parts by weight of lavender and 6 parts by weight of lemongrass. The floral mixture goes through extraction to obtain a blended essential oil; then the blended essential oil is diluted to 1000 times. The floral essential solution has the features of disinfection, sterilization, and skin benefits. It also has the ability to increase the amount of oxygen that the anion material can carry. The Chinese herbal essential layer 4 is a non-woven fabric layer soaked with the extracted Chinese herbal essential solution. The Chinese herbal essential solution is made from a mixture of Chinese herbs -containing 9 parts by weight of dried rehmannia root, 12 parts by weight of radix scrophulariae, 6 parts by weight of angelica, 12 parts by weight of honeysuckle flower, 9 parts by weight of forsythia fruit, 9 parts by weight of baicalin, 6 parts by weight of red paeony root, 9 parts by weight of tree paeony bark, 9 parts by weight of poria skin, 3 parts by weight of licorice, 10 parts by weight of Radix sophorae flavescentis, 15 parts by weight of common cnidium fruit, 15 pars by weight of belvedere fruit, 15 parts by weight of densefruit pittany root-bark, 6 parts by weight of pricklyash peel and 2 parts by weight of halite. The mixture of Chinese herbs is cooked, filtered, and then purified to obtain the Chinese herbal essential solution. The Chinese herbal essential solution has a remarkable beneficial effect on female' s outer genital itch, abnormal leucorrhea and vaginitis.

The far infrared anion fiber layer 3 is woven of fiber made from a mixture of 9 wt% of anion mother grain, 9 wt% of far infrared mother grain, and homopolymer carrier or spinning liquid. The anion mother grain is made from nano grade tourmaline micro-powder that activates anions in the air. The far infrared mother grain is made from nano grade far infrared ceramic micro-powder that activates far infrared. The far infrared anion fiber layer 3 can automatically release anion and far infrared.

The absorbent layer 5 is added with nano-silver and bamboo charcoal. The additives include 0.1 parts by weight of nano-silver having diameter less than 20nm, 1 part by weight of bamboo vinegar, and 1 part by weight of bamboo charcoal. The amount of the nano-silver adhered to the absorbent layer is 80pg/cm2.

The adhesive layer 7 is stripe interrupted adhesive layer, which improves airflow.

### Embodiment 2:

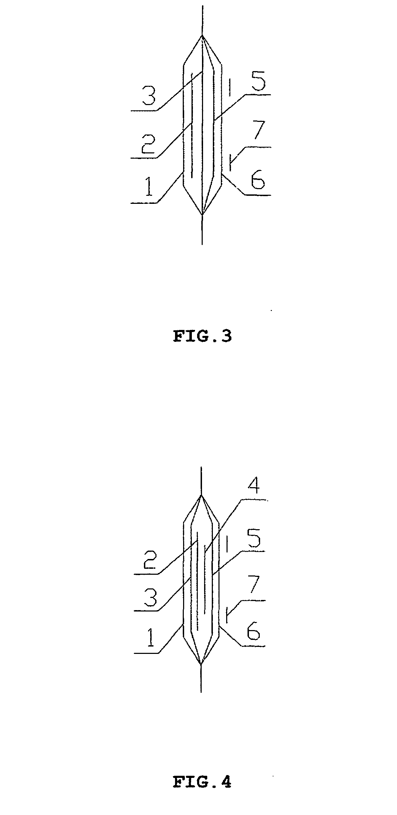

As shown in Fig. 3, the difference between this Embodiment and Embodiment 1 is the Chinese herbal essential layer 4 is omitted. Other configurations are substantially the same as in the sanitary napkin/pad of Embodiment 1.

### Embodiment 3:

As shown in Figs. 1 and 2, from upper to lower layer, the sanitary napkin/pad includes a web layer 1, a floral essential layer 2, a far infrared anion fiber layer 3, a Chinese herbal essential layer 4, an absorbent layer 5, a waterproof coating layer 6, and an adhesive layer 7. All layers are stuck firmly together.

The floral essential layer 2 is a puffy cotton layer that soaked with extracted floral essential solution. The floral essential solution is made from a floral mixture -of 3 parts by weight of lily, 8 parts by weight of honeysuckle flower, 3 parts by weight of chrysanthemum flower, 4 parts by weight of tree peony, 3 parts by weight of Chinese rose, 2 parts by weight of clove, 3 parts by weight of orange blossom, 1 part by weight of common coltsfoot flower, 3 parts by weight of lotus, 8 parts by weight of jasmine flower, 5 parts by weight of rose, 6 parts by weight of common peony, 3 parts by weight of silktree albizzia flower, 3 parts by weight of wintersweet flower, 5 parts by weight of peppermint, 4 parts by weight of licorice, 3 parts by weight of mugwort leaf, 4 parts by weight of drug sweetflag rhizome, 3 parts by weight of lavender, and 2 parts by weight of lemongrass. The mixture goes through extraction to obtain a blended essential oil; then the blended essential oil is diluted to 1200 times. The floral essential solution has the features of disinfection, sterilization, and skin benefits. It also has the ability to increase the amount of oxygen that the anion material can carry; the Chinese herbal essential layer 4 is puffy cotton layer soaked with extracted Chinese herbal essential solution. The Chinese herbal essential solution is made from a mixture of Chinese herbs containing 5 parts by weight of dried rehmannia root, 8 parts by weight of radix scrophulariae, 4 parts by weight of angelica, 8 parts by weight of honeysuckle flower, 5 parts by weight of forsythia fruit, 5 parts by weight of baicalin, 4 parts by weight of red paeony root, 5 parts by weight of tree paeony bark, 5 parts by weight of poria skin, 1 part by weight of licorice, 6 parts by weight of Radix sophorae flavescentis, 12 parts by weight of common cnidium fruit, 12 parts by weight of belvedere fruit, 12 parts by weight of densefruit pittany root-bark, 4 parts by weight of pricklyash peel, and 1 part by weight of halite. The mixture of Chinese herbs is cooked, filtered, and then purified to obtain the Chinese herbal essential solution. The Chinese herbal essential solution has remarkable beneficial effects on female's outer genital itch, abnormal leucorrhea, and vaginitis.

The far infrared anion fiber layer 3 is woven of fiber made from a mixture of 8 wt% of anion mother grain, 8 wt% of far infrared mother grain, and homopolymer carrier or spinning liquid. The anion mother grain is made from nano grade tourmaline micro-powder that activates anions in the air. The far infrared mother grain is made from nano grade far infrared ceramic micro-powder that activates far infrared. The far infrared anion fiber layer 3 can automatically release anion and far infrared.

The absorbent layer 5 is added with nano-silver and bamboo charcoal. The additives include 0.01 parts by weight of nano-silver having diameter less than 20nm and 0.1 parts by weight of bamboo charcoal. The amount of the nano-silver adhered to the absorbent layer is 5µg/cm2.

The adhesive layer 7 is stripe interrupted adhesive layer, whichi improves airflow.

### Embodiment 4:

As shown in Fig. 3, the difference between this Embodiment and Embodiment 3 is the Chinese herbal essential layer 4 is omitted. Other configurations are substantially the same as in the sanitary napkin/pad of Embodiment 3.

### Embodiment 5:

As shown in Figs. 1 and 2, from upper to lower layer, the sanitary napkin/padincludes a web layer 1, a floral essential layer 2, a far infrared anion fiber layer 3, a Chinese herbal essential layer 4, an absorbent layer 5, a waterproof coating layer 6, and an adhesive layer 7. All layers are stuck firmly together.

The floral essential layer 2 is a puffy cotton layer soaked with an extracted floral essential solution. The floral essential solution is made from a floral mixture of 8 parts by weight of lily, 14 parts by weight of honeysuckle flower, 8 parts by weight of chrysanthemum flower, 9 parts by weight of tree peony, 8 parts by weight of Chinese rose, 6 parts by weight of clove, 8 parts by weight of orange blossom, 6 part by weight of common coltsfoot flower, 8 parts by weight of lotus, 16 parts by weight of jasmine flower, 10 parts by weight of rose, 10 parts by weight of common peony, 8 parts by weight of silktree albizzia flower, 6 parts by weight of wintersweet flower , 12 parts by weight of peppermint, 10 parts by weight of licorice, 8 parts by weight of mugwort leaf, 8 parts by weight of drug sweetflag rhizome, 12 parts by weight of lavender, and 10 parts by weight of lemongrass. The floral mixture goes through extraction to obtain a blended essential oil; then the blended essential oil is diluted to 1100 times. The floral essential solution has the features of disinfection, sterilization, and skin benefits. It also has the ability to increase the amount of oxygen that the anion material can carry. The Chinese herbal essential layer 4 is a puffy cotton layer soaked with extracted Chinese herbal essential solution. The Chinese herbal essentialsolution is made from a mixture of Chinese herbs -containing 12 parts by weight of dried rehmannia root, 16 parts by weight of radix scrophulariae, 8 parts by weight of angelica, 16 parts by weight of honeysuckle flower,12 parts by weight of forsythia fruit, 12 parts by weight of baicalin, 12 parts by weight of red paeony root, 8 parts by weight of tree paeony bark, 12 parts by weight of poria skin, 5 part by weight of licorice, 15 parts by weight of Radix sophorae flavescentis, 18 parts by weight of common cnidium fruit, 18 parts by weight of belvedere fruit, 18 parts by weight of densefruit pittany root-bark, 8 parts by weight of pricklyash peel, and 4 parts by weight of halite. The mixture of Chinese herbs is cooked, filtered, and then purified to obtain the Chinese herbal essential solution. The Chinese herbal essential solution has remarkable beneficial effects on female's outer genital itch, abnormal leucorrhea, and vaginitis.

The far infrared anion fiber layer 3 is woven of fiber made from a mixture of 10 wt% of anion mother grain, 10 wt% of far infrared mother grain, and homopolymer carrier or spinning liquid. The anion mother grain is made of nano grade tourmaline micro-powder that activates anions in the air. The far infrared mother grain is made of nano grade far infrared ceramic micro-powder that activates far infrared. The far infrared anion fiber layer 3 can automatically release anion and far infrared.

The absorbent layer 5 is added with nano-silver and bamboo charcoal. The additives include 5 parts by weight of nano-silver having diameter less than 20nm, 10 parts by weight of bamboo vinegar, and 10 parts by weight of bamboo charcoal. The amount of the nano-silver adhered to the absorbent layer is 400µg/cm2.

The adhesive layer 7 is stripe interrupted adhesive layer, which improves airflow.

### Embodiment 6:

As shown in Fig 3, the difference between this Embodiment and Embodiment 5 is the Chinese herbal essential layer 4 is omitted. Other configurations are substantially the same as in the sanitary napkin/pad of Embodiment 5.

### Embodiment 7:

As shown in Fig. 4, the difference between this Embodiment and Embodiment 1 is: the far infrared anion complex sanitary napkin/pad comprises, in sequence from upper to lower layer, a web layer 1, a far infrared anion fiber layer 3, a floral essential layer 2, a Chinese herbal essential layer 4, an absorbent layer 5, a waterproof coating layer 6, and an adhesive layer 7.

### Embodiment 8:

As shown in Fig 3, the difference between this Embodiment and Embodiment 7 is the Chinese herbal essential layer 4 is omitted. Other configurations are substantially the same as in the sanitary napkin/pad of Embodiment 7.

Although the present invention has been described with reference to particular embodiment, it is not to be construed as being limited thereto. Various alterations and modifications can be made to the embodiment without in any way departing from the scope or spirit of the present invention.

## Claims

1. A far infrared anion complex sanitary napkin/pad having multi-layer structure, comprising, in sequence from upper to lower layer, a web layer, an absorbent layer, a waterproof coating layer and an adhesive layer stuck together, wherein the sanitary napkin/pad further comprises a floral essential layer and a far infrared anion fiber layer attached together firmly; the floral essential layer is a paper/fabric layer soaked with extracted floral essential solution; the far infrared anion fiber layer is woven of fiber formed from a mixture of anion mother grain, far infrared mother grain, and homopolymer carrier or spinning liquid; the anion mother grain is made from nano grade mineral powder capable of activating anion in the air; the far infrared mother grain is made from nano grade mineral powder capable of activating far infrared.

2. The far infrared anion complex sanitary napkin/pad according to Claim 1, wherein the nano grade mineral powder capable of activating anion in the air is nano grade tourmaline micro-powder, and the nano grade mineral powder capable of activating far infrared is nano grade far infrared ceramic micro-powder.

3. The far infrared anion complex sanitary napkin/pad according to Claim 1, wherein the absorbent layer is added with nano silver and bamboo vinegar or bamboo charcoal.

4. The far infrared anion complex sanitary napkin/pad according to Claim 1, wherein the sanitary napkin/pad further comprises a Chinese herbal essential layer, the Chinese herbal essential layer is a paper/fabric layer soaked with a Chinese herbal essential solution.

5. The far infrared anion complex sanitary napkin/pad according to Claim 1, 2 or 3, wherein the paper/fabric layer is a tissue layer, a non-woven fabric layer or a puffy cotton layer.

6. The far infrared anion complex sanitary napkin/pad according to Claim 5, wherein the floral essential layer is above the Chinese herbal essential layer.

7. The far infrared anion complex sanitary napkin/pad according to Claim 6, wherein the floral essential layer is attached to the far infrared anion fiber layer firmly.

8. The far infrared anion complex sanitary napkin/pad according to Claim 7, wherein the floral essential layer is above the far infrared anion fiber layer.

9. The far infrared anion complex sanitary napkin/pad according to Claim 8, wherein the Chinese herbal essential layer is located at the central portion of the sanitary napkin/pad, and the ratio of the length and the width of the Chinese herbal essential layer with respect to the length and the width of the sanitary napkin/pad is in a range of 1/2 to 2/3.

10. The far infrared anion complex sanitary napkin/pad according to Claim 1 or 2, wherein the floral essential layer is above the Chinese herbal essential layer.

## Patentansprüche

1. Ferninfrarot-Anionenkomplex-Damenbinde/-Auflage, die eine mehrlagige Struktur aufweist und in der Reihenfolge von der obersten bis zur untersten Lage eine Netzschicht, eine absorbierende Schicht, eine wasserundurchlässige Beschichtungslage und eine Haftschicht umfasst, die miteinander verklebt sind, wobei die Damenbinde/Auflage weiterhin eine Blumenessenzschicht und eine Ferninfrarot-Anionen-Faserschicht umfasst, die fest miteinander verbunden sind; die Blumenessenzschicht ist eine Papier-/Gewebe-Schicht, die mit einer Extraktionslösung aus Blumenessenz getränkt ist; die Ferninfrarot-Anionen-Faserschicht wird aus Fasern gewebt, die aus einer Mischung eines Anionen-Masterbatch, eines Ferninfrarot-Masterbatch und einer Homopolymerträgerflüssigkeit oder einer rotierenden Homopolymerflüssigkeit gebildet werden; das Anionen-Masterbatch wird aus einem nanoskaligen Mineralpulver hergestellt, das Anionen in der Luft aktivieren kann; das Ferninfrarot-Masterbatch wird aus einem nanoskaligen Mineralpulver hergestellt, das Ferninfrarot aktivieren kann.

2. Ferninfrarot-Anionenkomplex-Damenbinde/-Auflage nach Anspruch 1, wobei das nanoskalige Mineralpulver, das Anionen in der Luft aktivieren kann, ein nanoskaliges Turmalinmikropulver ist und das nanoskalige Mineralpulver, das Ferninfrarot aktivieren kann, ein nanoskaliges Ferninfrarot-Keramikmikropulver ist.

3. Ferninfrarot-Anionenkomplex-Damenbinde/-Auflage nach Anspruch 1, wobei der absorbierenden Schicht Nanosilber und Bambusessig oder Bambusaktivkohle beigefügt werden.

4. Ferninfrarot-Anionenkomplex-Damenbinde/-Auflage nach Anspruch 1, wobei die Damenbinde/Auflage weiterhin eine chinesische Kräuteressenzschicht umfasst und die chinesische Kräuteressenzschicht eine Papier-/Gewebe-Schicht ist, die mit einer Lösung aus chinesischer Kräuteressenz getränkt ist.

5. Ferninfrarot-Anionenkomplex-Damenbinde/-Auflage nach Anspruch 1, 2 oder 3, wobei die Papier-/Gewebe-Schicht eine Zellstoffschicht, eine Faservliesschicht oder eine Baumwollwatteschicht ist.

6. Ferninfrarot-Anionenkomplex-Damenbinde/-Auflage nach Anspruch 5, wobei sich die Blumenessenzschicht oberhalb der chinesischen Kräuteressenzschicht befindet.

7. Ferninfrarot-Anionenkomplex-Damenbinde/-Auflage nach Anspruch 6, wobei die Blumenessenzschicht fest mit der Ferninfrarot-Anionen-Faserschicht verbunden ist.

8. Ferninfrarot-Anionenkomplex-Damenbinde/-Auflage nach Anspruch 7, wobei sich die Blumenessenzschicht oberhalb der Ferninfrarot-Anionen-Faserschicht befindet.

9. Ferninfrarot-Anionenkomplex-Damenbinde/-Auflage nach Anspruch 8, wobei die chinesische Kräuteressenzschicht am zentralen Abschnitt der Damenbinde/Auflage angeordnet ist und das Verhältnis der Länge und Breite der chinesischen Kräuteressenzschicht gegenüber der Länge und Breite der Damenbinde/Auflage in einem Bereich zwischen 1/2 bis 2/3 liegt.

10. Ferninfrarot-Anionenkomplex-Damenbinde/-Auflage nach Anspruch 1 oder 2, wobei sich die Blumenessenzschicht oberhalb der chinesischen Kräuteressenzschicht befindet.

## Revendications

1. Serviette hygiénique/couche contenant un complexe anionique dans l'infrarouge lointain ayant une structure multicouche, comprenant, en séquence de la couche supérieure à la couche inférieure, une couche centrale, une couche absorbante, une couche à revêtement imperméable à l'eau et une couche adhésive collées ensemble, ladite serviette hygiénique/couche comprenant en outre une couche d'essence florale et une couche de fibre anionique dans l'infrarouge lointain fixées fermement ensemble ; la couche d'essence florale est une couche de papier/tissu imprégnée d'extrait de solution essentielle florale ; la couche de fibre anionique dans l'infrarouge lointain est tissée à partir de fibres formées à partir d'un mélange de grain mère anionique, de grain mère dans l'infrarouge lointain et d'un support homopolymère ou un liquide de filage ; le grain mère anionique est réalisé à partir d'une poudre minérale de l'ordre des nanoparticules capable d'activer l'anion dans l'air ; le grain mère dans l'infrarouge lointain est réalisé à partir d'une poudre minérale de l'ordre des nanoparticules capable d'activer l'infrarouge lointain.

2. Serviette hygiénique/couche contenant un complexe anionique dans l'infrarouge lointain selon la revendication 1, dans laquelle la poudre minérale de l'ordre des nanoparticules capable d'activer l'anion dans l'air est de la micropoudre de tourmaline de l'ordre des nanoparticules, et la poudre minérale de l'ordre des nanoparticules capable d'activer l'infrarouge lointain est de la micropoudre de céramique dans l'infrarouge lointain de l'ordre des nanoparticules.

3. Serviette hygiénique/couche contenant un complexe anionique dans l'infrarouge lointain selon la revendication 1, dans laquelle la couche absorbante est additionnée de nanoparticules d'argent et de vinaigre de bambou ou de charbon de bois de bambou.

4. Serviette hygiénique/couche contenant un complexe anionique dans l'infrarouge lointain selon la revendication 1, ladite serviette hygiénique/couche comprenant en outre une couche d'essence de plantes médicinales chinoises, la couche d'essence de plantes médicinales chinoises est une couche de papier/tissu imprégnée avec une solution essentielle de plantes médicinales chinoises.

5. Serviette hygiénique/couche contenant un complexe anionique dans l'infrarouge lointain selon la revendication 1, 2 ou 3, dans laquelle la couche de papier/tissu est une couche de tissu, une couche de tissu non tissé ou une couche de coton gonflant.

6. Serviette hygiénique/couche contenant un complexe anionique dans l'infrarouge lointain selon la revendication 5, dans laquelle la couche d'essence florale est au-dessus de la couche d'essence de plantes médicinales chinoises.

7. Serviette hygiénique/couche contenant un complexe anionique dans l'infrarouge lointain selon la revendication 6, dans laquelle la couche d'essence florale est fermement fixée à la couche de fibre anionique dans l'infrarouge lointain.

8. Serviette hygiénique/couche contenant un complexe anionique dans l'infrarouge lointain selon la revendication 7, dans laquelle la couche d'essence florale est au-dessus de la couche de fibre anionique dans l'infrarouge lointain.

9. Serviette hygiénique/couche contenant un complexe anionique dans l'infrarouge lointain selon la revendication 8, dans laquelle la couche d'essence de plantes médicinales chinoises est positionnée au niveau de la partie centrale de la serviette hygiénique/couche, et le rapport de la longueur et de la largeur de la couche d'essence de plantes médicinales chinoises par rapport à la longueur et à la largeur de la serviette hygiénique/couche est de l'ordre de 1/2 à 2/3.

10. Serviette hygiénique/couche contenant un complexe anionique dans l'infrarouge lointain selon la revendication 1 ou 2, dans laquelle la couche d'essence florale est au-dessus de la couche d'essence de plantes médicinales chinoises.
